# EUROPEAN PATENT SPECIFICATION

(11) **EP 3 439 743 B1**
(45) Date of publication and mention of the grant of the patent: **20.11.2019**
(21) Application number: 16716472.2
(22) Date of filing: 07.04.2016
(51) Int. Cl.: A61Q 19/00, A61Q 19/02, A61Q 19/08, A61K 8/97

(54) **COMPOSITION COMPRISING HOUSELEEK (SEMPERVIVUM TECTORUM L.) EXTRACT, PREPARATION PROCESSES AND USES THEREOF**
ZUBEREITUNG ENTHALTEND HAUSWURZEXTRAKT (SEMPERVIVUM TECTORUM L.), VORBEREITUNG UND VERWENDUNGEN
COMPOSITION COMPRENANT L'EXTRACT DE JOUBARBE (SEMPERVIVUM TECTORUM L.), SA PREPARATION ET SON UTILISATION

(43) Date of publication of application: 13.02.2019
(73) Proprietor: Botanica GmbH, 5643 Sins (CH)
(72) Inventor: WÄLTI, Andreas, 8032 Zürich (CH)
(74) Representative: Feldmann, Ute
(86) International application number: PCT/EP2016/000577
(87) International publication number: WO 2016/177443

(56) References cited:
- CN-A- 1 579 456
- DE-A1- 19 922 385
- DATABASE WPI Week 200916 1 February 2008 (2008-02-01) Thomson Scientific, London, GB; AN 2009-F24804 XP002762761, & TW 200 806 337 A (LI S) 1 February 2008 (2008-02-01)

## Description

### Technical Field:

The present invention relates to a non-therapeutic use of a houseleek (*sempervivum tectorum L.*) extract in a cosmetic formulation, characterized in that the cosmetic formulation is used for enhancing an anti-ageing process in skin, enhancing an anti-wrinkle process in skin, for enhancing a refirming process in skin, for enhancing an anti-cellulite process in skin, for enhancing a regeneration process in skin, for enhancing skin lightening and/or skin whitening and/or for enhancing skin condition of sensitive skin as well as a corresponding cosmetic formulation, a process of preparation of an inventive houseleek (*sempervivum tectorum L.*) extract and a houseleek (*sempervivum tectorum L*.) extract obtainable in accordance with the inventive preparation process.

### Background:

The cosmetic industry already provides cosmetic formulations for different application fields. One application field concerns enhancing an anti-ageing process in skin, enhancing an anti-wrinkle process in skin, refirming skin, enhancing an anti-cellulite process in skin, and/or regenerating skin.

A further application field concerns the provision of skin lightening / whitening products.

Another application field correlates with the provision of skin care products applicable for sensitive skin.

Due to the present "green" marketing trend there exists a need of providing "organic" or "natural" cosmetic alternatives suitable for above mentioned applications.

Houseleek of the kind *sempervivum tectorum L.* (*e.g.* syn.: *sempervivum alpinum* or *sempervivum schottii*) was first described by Linnaeus in 1753 and is a rosette-forming succulent, evergreen and perennial plant, which spreads by offsets. Historically, houseleek leaves were used as a medicinal herb for diverse treatments such as external treatment of skin injuries, for internal treatment of throat infections, uterine neuralgia, dysplasia and amenorrhea. However, the effectiveness of the drug has not been proven in the mentioned application areas. Modern research confirmed the effectiveness of houseleek juice for treating ear pain due to antimicrobial activity towards clinical isolates of bacteria linked to otitis (Soikovic D et al, "Ethnopharmacological uses of Sempervivum tectorum L in southern Serbia: Scientific confirmation for the use against otitis linked bacteria", J Ethnopharmacol. 2015 Dec 24; 176:297-304). Additionally, houseleek is used as a food product, in particular as spice or salad.

Accordingly, there exists an aim to provide alternative cosmetic skin care products with comparative and/or improved cosmetic effects on
❖ anti-ageing and/or
❖ regenerating skin and/or
❖ anti-wrinkle
❖ refirming skin and/or
❖ enhancing an anti-cellulite process in skin and/or
❖ skin lightening and/or
❖ treating sensitive skin.

### Brief Description of the Invention:

The aforementioned aims are solved at least in part solved by means of the claimed inventive subject matter. Preferred embodiments are described in the dependent claims as well as in the following description.

Accordingly a first aspect of the invention relates to a non-therapeutic use of a houseleek (*sempervivum tectorum L.*) extract, preferably houseleek (*sempervivum tectorum L.*) leaf extract in a cosmetic formulation, characterized in that the houseleek (*sempervivum tectorum L.*) extract is used for enhancing an anti-ageing process in skin, enhancing an anti-wrinkle process in skin, for enhancing a refirming process in skin, for enhancing an anti-cellulite process in skin, for enhancing a regeneration process in skin, and/or for enhancing skin lightening and/or skin whitening.

A second aspect of the invention relates to a cosmetic formulation for use in enhancing skin condition of sensitive skincomprising
a) an effective amount of houseleek (*sempervivum tectorum L.)* extract, preferably houseleek (*sempervivum tectorum L.*) leaf extract for enhancing skin condition of sensitive skin and
b) one, two, three or more cosmetically acceptable carriers, excipients and/or additives.

A third aspect of the invention relates to a process of preparing a houseleek (*sempervivum tectorum L.*) extract, preferably houseleek (*sempervivum tectorum L.*) leaf extract for use in cosmetic formulations, characterized in that the process comprises or consists of the following steps:
a) providing a suitable amount of houseleek (*sempervivum tectorum L.*)*,* preferably, houseleek (*sempervivum tectorum L.*) leaves,
b) providing a suitable amount of extraction agent,
c) forming an extraction phase by combining the houseleek of step a) with the extraction agent of step b), extracting the houseleek and providing the houseleek (*sempervivum tectorum L.*) extract, preferably providing the houseleek (*sempervivum tectorum L.*) leaf extract
d) optionally separating by suitable means at least part of the remaining solid content of the houseleek (*sempervivum tectorum L.*) from the extraction phase of step c) after finalizing the extraction,
e) optionally evaporating by suitable means at least part of the extraction agent in step c) after finalizing the extraction to produce a houseleek (*sempervivum tectorum L.*) extract concentrate and
f) adding a suitable amount of a storing additive agent selected from the group consisting of glycerin, propylene glycole, polysorbate, sorbitole, hydrogenated castor oil, or pentylene glycole, to the produced houseleek (*sempervivum tectorum L.*) extract of step c) or d) or houseleek (*sempervivum tectorum L.*) extract concentrate of step e).

A fourth aspect of the invention relates to a houseleek (*sempervivum tectorum L.*) extract, preferably houseleek (*sempervivum tectorum L.*) leaf extract obtainable in accordance with the inventive preparation process.

The aforementioned embodiments of the inventive aspects can - as far it is reasonable in view of a technical expert - comprise any possible combination of the preferred inventive embodiments, which are disclosed in the following and in particular in the dependent claims.

### Brief Description of Drawings:

**Fig. 1a****)** shows a graph on effects of activation of Peroxisome Proliferator-Activated Receptor α (PPARα) in 3T3-L1 adipocyte cells by the inventive houseleek (*sempervivum tectorum L.*) extract.
**Fig. 1b****)** shows a graph on effects of activation of Peroxisome Proliferator-Activated Receptor γ (PPARγ) in 3T3-L1 adipocyte cells by the inventive houseleek (*sempervivum tectorum L.*) extract.
**Fig. 2** shows a graph on effects on inhibition of Transient receptor Potential cation channel subfamily V member 1 (TrPV1) by the inventive houseleek (*sempervivum tectorum L.*) extract vs. positive control of 10 µM Capsazepine.
**Fig. 3** shows a graph on effects of increased glucose uptake in keratinocytes by the inventive houseleek (*sempervivum tectorum L.*) extract vs. positive control of 80 mM Rosiglitazone.
**Fig. 4a****)** shows a graph on effects of inhibition of Hypoxia-Inducible Factor 1α (HIF-1α) activity in fibroblasts by the inventive houseleek (*sempervivum tectorum L.*) extract vs. positive control of 150 µM hypoximimetic DFX.
**Fig. 4b****)** shows a graph on effects of inhibition of Hypoxia-Inducible Factor 1α (HIF-1α) activity in keratinocytes by the inventive houseleek (*sempervivum tectorum L.*) extract vs. positive control of 150 µM hypoximimetic DFX.
**Fig. 5** shows a graph on effects of inhibition of tyrosinase activity in B16 melanoma cells by the inventive houseleek (*sempervivum tectorum L.*) extract vs. positive control of 2 mM Kojic Acid.
**Fig. 6** shows a graph on effects of inhibition of melanin synthesis activity in B16 melanoma cells by the inventive houseleek (*sempervivum tectorum L.*) extract vs. positive control of 2 mM Kojic Acid.
**Fig. 7** shows a graph on effects of activation of CannaBinoid receptor type 1 (CB 1) in 293T-CB1 cells by the inventive houseleek (*sempervivum tectorum L.*) extract vs. positive control of 1 µM WIN55,212-2.

### Detailed Description of the Invention:

According to the aspects of the present invention, the inventor has surprisingly found out that the inventive houseleek (*sempervivum tectorum L.*) extract, preferably houseleek (*sempervivum tectorum L.*) leaf extract, in particular obtainable by the inventive manufacturing process exhibits one, two, three, four, five, six or seven or more comparative and/or improved cosmetic effects on
❖ anti-ageing and/or
❖ regenerating skin and/or
❖ anti-wrinkle
❖ refirming skin and/or
❖ enhancing an anti-cellulite process in skin and/or
❖ skin lightening and/or
❖ treating sensitive skin.

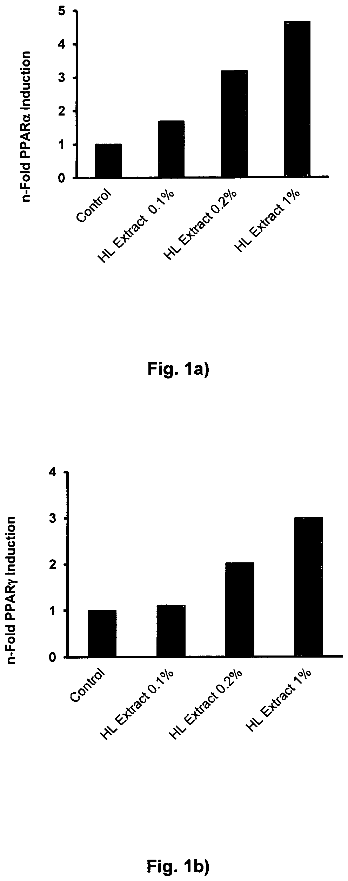

The effectiveness in the uses of the first and second aspect of the present invention has been proven by conducting the biologic experiments set out in the example section, in particular under part B as well as in Figures 1a), 1b), 2, 3, 4a), 4b), 5, 6, and 7.

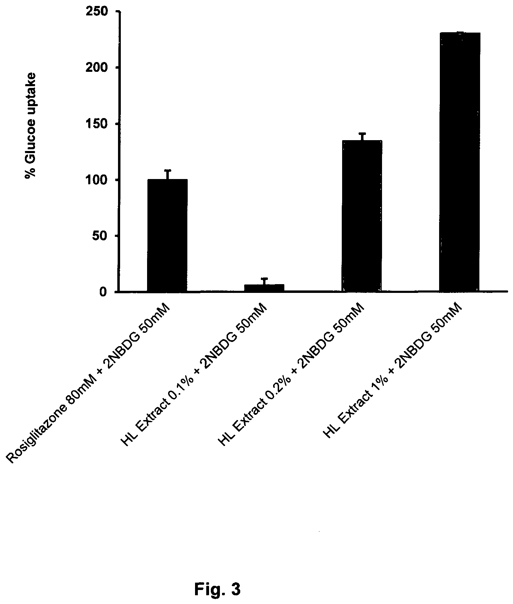

The anti-ageing and regenerating effects are, e.g., proved by the stimulating effect of the inventive houseleek (*sempervivum tectorum L.*) extract, preferably houseleek (*sempervivum tectorum L.*) leaf extract on the glucose uptake in keratinocytes (see experimental section, part B.3 and Figure 3). Glucose lays a vital role in the energy metabolism of keratinocytes and is in particular correlated with cell regeneration. Thus, the inventive houseleek (*sempervivum tectorum L.*) extract, preferably houseleek (*sempervivum tectorum L.*) leaf extract seems to be useful in reversing aged skin condition and promoting regenerated skin. Furthermore, the activation of Peroxisome Proliferator-Activated Receptor α (PPARα) in 3T3-L1 adipocyte cells by the inventive houseleek (*sempervivum tectorum L.*) extract, preferably houseleek (*sempervivum tectorum L.*) leaf extract (see experimental section, part B.1 and Figure 1a) also proves a positive effect on anti-ageing and regeneration, as PPARα agonists activate β-oxidation of fatty acids, seem to play a role in cell differentiation and proliferation. Both, the increased glucose uptake into keratinocytes as well as the activation of PPARα may also contribute to an improved moisturizing effect at the same time.

Skin regeneration relates to the treatment of functional skin cells and the skin regeneration processes can in particular comprise (in no particular order):
❖ Skin aging processes can be stopped or reduced
❖ Collagen and hyaluronic acid synthesis are revived
❖ Tissue production rises
❖ Skin elasticity and skin tightness increase
❖ Skin becomes thicker
❖ Mechanisms that support skin protection activate again
❖ Decay is slowed down
❖ Toxic substances are degraded
❖ Metabolic processes are stimulated
❖ Increased glucose uptake stimulates cell metabolism
❖ Skin hydration increases

In contrast, skin healing refers to a restoration of health to a damaged or diseased skin cells to become functional again. Damaged or necrotic skin areas are repaired or reduced in size by cells in our body, which work in a reparative fashion to provide new living tissue to those areas. Replacement is usually performed in two ways - on the one hand, necrotic skin cells can be replaced with new cells that form similar tissue or, on the other hand, by repair, meaning injured skin tissue is replaced with scar tissue. Usually, healing is a combination of both mechanisms.

Thus, the effect of skin regeneration is not the same as healing of damaged skin, e.g., by burns etc.

The anti-wrinkle and refirming effects are, e.g., proved by the activation of Peroxisome Proliferator-Activated Receptor γ (PPARγ) in 3T3-L1 adipocyte cells by the inventive houseleek (*sempervivum tectorum L.*) extract, preferably houseleek (*sempervivum tectorum L.*) leaf extract (see experimental section, part B.1 and Figure 1b). PPARγ agonists activate cell development (proliferation) and lipogenesis and lead to an increased collagen production in keratinocytes, which result in refirming and anti-wrinkle effects.

Additionally, the inhibition of Transient receptor Potential cation channel subfamily V member 1 (TrPV1) by the inventive houseleek (*sempervivum tectorum L.*) extract, preferably houseleek (*sempervivum tectorum L.*) leaf extract vs. positive control of 10 µM Capsazepine as described in the example section in part B.3 and Figure 3. TrPV1 may prove the anti-ageing, regenerating, refirming and ant-wrinkle effects, as TrPV1 inhibitors are described to promote anti-ageing effects, wrinkle improvement and skin whitening.

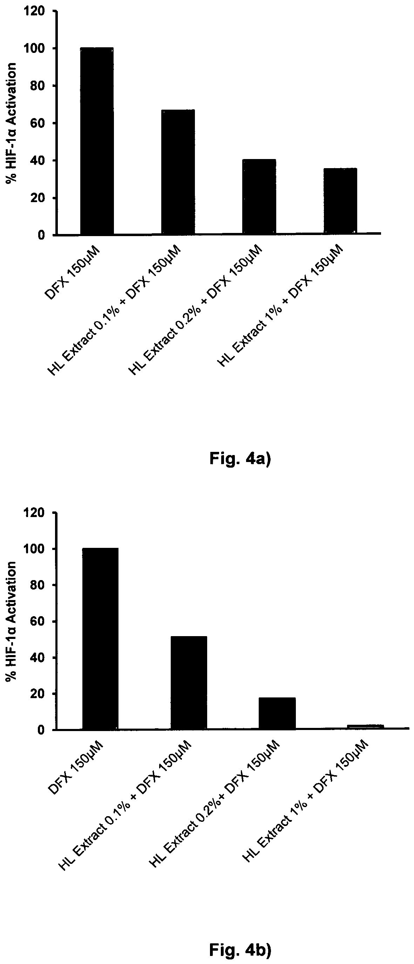

The inhibition of Hypoxia-Inducible Factor 1α (HIF-1α) activity in fibroblasts by the inventive houseleek (*sempervivum tectorum L.*) extract, preferably houseleek (*sempervivum tectorum L.*) leaf extract vs. positive control of 150 µM hypoximimetic DFX may furthermore prove the refirming and anti-wrinkle effects, as HIF-1α regulates cutaneous angiogenesis (see example section, part B.4 and Figures 4a) and 4b)).

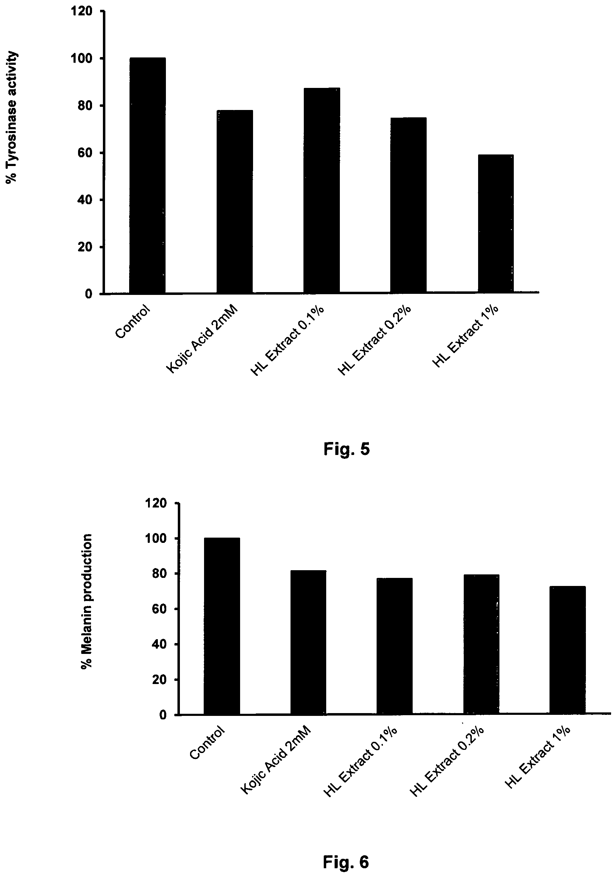

Positive effects on skin lightening and/or skin whitening for the inventive houseleek (*sempervivum tectorum L.*) extract, preferably houseleek (*sempervivum tectorum L.*) leaf extract are proved by the inhibition of tyrosinase activity in B16 melanoma cells by the inventive houseleek (*sempervivum tectorum L.*) extract, preferably houseleek (*sempervivum tectorum L.*) leaf extract vs. positive control of 2 mM Kojic Acid (see example section, part B.5, Figure 5) as well as the inhibition of melanin synthesis activity in B16 melanoma cells by the inventive houseleek (*sempervivum tectorum L.*) extract, preferably houseleek (*sempervivum tectorum L.*) leaf extract vs. positive control of 2 mM Kojic Acid (see example section, part B.6 and Figure 6).

Treating sensitive skin is one of the most important areas of dermatology. According to a number of studies, about 50% of the population claim to suffer from sensitive skin. Sensitivity means that the skin can easily be stressed by different extrinsic and intrinsic factors. People complain about several skin irritations that feel like burning, itching and/or stinging. Sensitive skin occurs irrelevant of the skin condition like dry, normal or fatty skin condition.

Research has identified different mechanisms which play a role for sensitive skin. Cannabinoid receptor type 1 and TRP channels are two important ones.

Cannabinoid signaling is mentioned for regulating the permeability barrier and epidermal differentiation. Cannabinoid receptor type 1 in keratinocytes help to maintain epidermal barrier homoeostasis and attenuate Th2-type allergic inflammatory responses. A study by Ständer et al. about cannabinoid agonists mention a mast cell stabilizing function which helps against itching and inhibits histamine secretion.

TRP channels are stimulated by external environmental factors like UV light, cold, heat, air pollution and chemical substances. They play a role in the recognition and physiology of sensitive skin. TrPV1, is among other stimuli activated by capsaicin. TrPV1 inhibitors are described to have skin-aging prevention, wrinkle improvement and skin-whitening features, and seem to alleviate inflammation, irritation or pain.

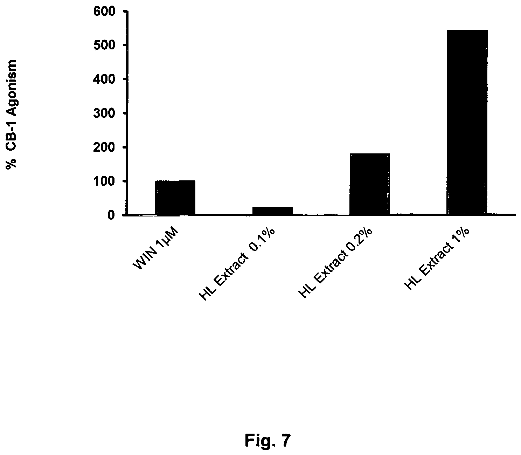

The activation of CannaBinoid receptor type 1 (CB 1) in 293T-CB1 cells by the inventive houseleek (*sempervivum tectorum L.*) extract, preferably houseleek (*sempervivum tectorum L.*) leaf extract vs. positive control of 1 µM WIN55,212-2 proves the ability to treat sensitive skin by the inventive houseleek (*sempervivum tectorum L.*) extract, preferably houseleek (*sempervivum tectorum L.*) leaf extract (see example section, part B.7 and Figure 7). CB 1 agonists help to maintain epidermal barrier homesostastis and stabilize mast cell stabilizing function, which helps against itching and inhibits histamine secretion.

In contrast to sensitive skin, dry skin is acknowledged in the art as the loss of water retention in the stratum corneum (SC). Water is essential for several skin functions. The retention of water in the SC is dependent on two major components: the presence of natural hygroscopic agents within the corneocytes (collectively referred to as natural moisturizing factor) and the SC intercellular lipids orderly arranged to form a barrier to transepidermal water loss (TEWL).

The inventive houseleek (*sempervivum tectorum L.*) extract, preferably houseleek (*sempervivum tectorum L.*) leaf extract is an agonist of cannabinoid receptor type 1 and shows antagonistic activity against TrPV1. Consequently it can be seen as effective in treatment for sensitive skin.

According to the present invention the term *"houseleek"* and/or *"houseleek* (*sempervivum tectorum L.*)*"* are used synonymously and cover *sempervivum tectorum L..* In a preferred embodiment of the present invention the term *"houseleek'* and/or *"houseleek* (*sempervivum tectorum L*)*"* is limited to *sempervivum tectorum L.* or its synonym forms.

According to the present invention the term *"houseleek extract"* and/or *"houseleek (sempervivum tectorum L.) extract"* comprises the *"houseleek extract concentrate"* and *"houseleek (sempervivum tectorum L.) extract concentrate"* as well as a preferred embodiment the *"houseleek glycerin extract concentrate"* and *"houseleek (sempervivum tectorum L.) glycerin extract concentrate".* In other word, in case the term *"houseleek extract"* and/or *"houseleek (sempervivum tectorum L.) extract"* is used in the present application the further embodiments of the *"houseleek extract concentrate"* and *"houseleek (sempervivum tectorum L.) extract concentrate"* as well as a preferred embodiment the *"houseleek glycerin extract concentrate"* and *"houseleek (sempervivum tectorum L.) glycerin extract concentrate"* are also disclosed therewith. The inventive *"houseleek extract"* and/or *"houseleek (sempervivum tectorum L.) extract"* can prepared in liquid, concentrated or solid (e.g., dried, freeze-dried) form.

According to the present invention, all parts of the inventive houseleek can be used for preparing the inventive *"houseleek extract"* and/or *"houseleek (sempervivum tectorum L.) extract".* According to a preferred embodiment of the invention the upper part of the houseleek plant, in particular the leaves and optionally part of the stem or stalks is present when preparing the inventive *"houseleek extract"* and/or *"houseleek (sempervivum tectorum L.) extract".* Accordingly, the following names may be used in the present application for the inventive houseleek extracts: *"houseleek leaf extract"* and/or *"houseleek (sempervivum tectorum L.) leaf extract"* and/or *"houseleek leaf extract concentrate"* and/or *"houseleek (sempervivum tectorum L.) leaf extract concentrate"* and/or *"houseleek leaf glycerin extract concentrate"* and/or *"houseleek (sempervivum tectorum L.) leaf glycerin extract concentrate".*

According to the second aspect of the present invention, the inventive *"houseleek extract"* and/or *"houseleek (sempervivum tectorum L.) extract",* preferably as the inventive *"houseleek leaf extract"* and/or *"houseleek (sempervivum tectorum L.) leaf extract"* can be used in a cosmetic formulation comprising one, two, three or more cosmetically acceptable carriers, excipients and/or additives for enhancing an anti-ageing process in skin, enhancing an anti-wrinkle process in skin, for enhancing a refirming process in skin, for enhancing an anti-cellulite process in skin, for enhancing a regeneration process in skin, for enhancing skin lightening and/or skin whitening and/or for enhancing skin condition of sensitive skin. As one, two, three or more cosmetically acceptable carriers, excipients and/or additives generally suitable cosmetically acceptable carriers, excipients and/or additives may be used for the second inventive aspects. Suitable cosmetic carriers, excipients and/or additives are in particular disclosed in "actifs et additifs en cosmétologie" (3e edition, Marie-Claude Martini et Monique Seiller, Lavoisier 2006 ISBN 978-2-7430-0711-9). The disclosure in "actifs et additifs en cosmétologie" concerning cosmetic carriers, excipients and/or additives are incorporated herein by reference.

According to the second aspect of the present invention, the inventive houseleek (*sempervivum tectorum L.*) extract, the inventive houseleek (*sempervivum tectorum L.*) extract concentrate and / or the inventive houseleek (*sempervivum tectorum L.*) glycerin extract concentrate, preferably the houseleek (*sempervivum tectorum L.*) leaf extract, the inventive houseleek (*sempervivum tectorum L.*) leaf extract concentrate and / or the inventive houseleek (*sempervivum tectorum L.*) leaf glycerin extract concentrate can be added to any suitable cosmetic formulation. The suitable cosmetic formulation can be selected from the group consisting of liquid, solid and half solid skin care formulations, preferably wherein the cosmetic formulations are selected from the group of an ointment, creme, lotion, gel, sun screen product (e.g., creme, lotion, gel), after sun screen product (e.g., creme, lotion, gel), tincture, solution (e.g., serum, fluid), oil product (e.g., oil as skin care, oil bath), soap, cleansing product, shampoo, powder product, makeup, spray, bath salt, (skin)masks, deodorant, towelett, butter, balm, exfoliant, moisturizer, toner.

The inventive houseleek (*sempervivum tectorum L.*) extract, preferably houseleek (*sempervivum tectorum L.*) leaf extract is generally added in an effective amount to a suitable cosmetic formulation in accordance with the second aspect of the present invention to enhance
❖ an anti-ageing process in skin and/or
❖ an anti-wrinkle process in skin and/or
❖ a refirming process in skin and/or
❖ an anti-cellulite process in skin and/or
❖ a regeneration process of skin and/or
❖ a skin lightening and/or skin whitening process and/or
❖ skin condition of sensitive skin.

In a preferred embodiment, the inventive cosmetic formulation of the second aspect of the invention is used to enhance an anti-ageing process in skin and a regeneration process of skin and/or an anti-wrinkle process in skin and a refirming process in skin and/or an anti-cellulite process in skin and/or a skin lightening and/or skin whitening process and/or skin condition of sensitive skin. More preferably, the inventive cosmetic formulation of the second aspect of the invention is used to enhance sensitive skin condition and an anti-ageing process in skin and a regeneration process of skin and/or sensitive skin condition and an anti-wrinkle process in skin and a refirming process in skin and/or sensitive skin condition and an anti-cellulite process in skin and/or sensitive skin condition and a skin lightening and/or skin whitening process. Alternatively, the inventive cosmetic formulation of the second aspect of the invention is used to enhance an anti-ageing process in skin and a regeneration process of skin and an anti-wrinkle process in skin and a refirming process in skin and an anti-cellulite process in skin and a skin lightening and/or skin whitening process.

In general, the respective amounts of the inventive houseleek (*sempervivum tectorum L.*) extract, the inventive houseleek (*sempervivum tectorum L.*) extract concentrate and/or the inventive houseleek (*sempervivum tectorum L.*) glycerin extract concentrate, preferably the inventive houseleek *(sempervivum tectorum L.*) leaf extract, the inventive houseleek (*sempervivum tectorum L.*) leaf extract concentrate and/or the inventive houseleek (*sempervivum tectorum L.*) leaf glycerin extract concentrate can range - dependent from use - between approximately 0.1 wt.-% to 95 wt.-%, alternatively 0.2 wt.-% to 80 wt.-% alternatively 0.3 wt.-% to 60 wt.-%, alternatively 0.4 wt.-% to 40 wt.-%, alternatively 0.5 wt.-% to 20 wt.-%, alternatively 0.6 wt.-% to 15 wt.-%, alternatively 0.7 wt.-% to 10 wt.-%, alternatively 0.8 wt.-% to 9 wt.-%, alternatively 0.9 wt.-% to 8 wt.-%, alternatively 1 wt.-% to 7 wt.-%, alternatively 1.1 wt.-% to 6 wt.-%, alternatively 1.2 wt.-% to 5 wt.-%, alternatively 1.3 wt.-% to 4 wt.-%, alternatively 1.4 wt.-% to 3 wt.-%, alternatively 1.5 wt. to 2 wt.-% based on the total weight of the inventive cosmetic formulation of the second aspect of the present invention.

Preferably, the inventive *"houseleek extract"* and/or *"houseleek* (*sempervivum tectorum L.*) *extract",* more preferably the inventive *"houseleek leaf extract"* and/or *"houseleek* (*sempervivum tectorum L.*) *leaf extract"* used in the second aspect of the present invention is obtainable by the inventive preparation process of the third aspect of the present invention.

According to the third aspect of the present invention the inventive preparation process of the inventive houseleek extract and/or houseleek (*sempervivum tectorum L.*) extract, preferably the inventive houseleek leaf extract and/or houseleek (*sempervivum tectorum L.*) leaf extract is generally conducted in the following way:
a: A suitable amount of houseleek (*sempervivum tectorum L.*)*,* preferably of cut houseleek (*sempervivum tectorum L.*) in particular mainly comprising houseleek leaves, is provided in a suitable extraction vessel, e.g. a suitable flask.
   In the present example 1 kg dried and cut houseleek (*sempervivum tectorum L.*) leaves, BIO CH are weighed out into a suitable flask for facilitating extraction of the cut houseleek leaves.
b: A suitable amount of extraction agent is provided.
   As suitable extraction agent, e.g., any suitable hydrophilic extraction agent can be used, such as water and/or one, two or more suitable extraction alcohols, e.g., ethanol, isopropanol, propanol, n-butanol, isobutanol etc.. In the present example a suitable amount of ethanol (94 wt.-%) is used as the extraction agent.
c: The (dried cut) houseleek (*sempervivum tectorum L.*)*,* preferably houseleek (*sempervivum tectorum L.*) leaves of step a) are extracted by adding of the suitable extraction agent of step b) into the suitable extraction vessel of step a) forming an extraction phase and extracting the houseleek to form the inventive houseleek extract and/or houseleek (*sempervivum tectorum L.*) extract, preferably the inventive houseleek leaf extract and/or houseleek (*sempervivum tectorum L.*) leaf extract.
   A suitable amount of suitable extraction agent is added to the dried cut houseleek (*sempervivum tectorum L.*)*,* preferably mainly comprising leaves of step a) to at least partly, preferably fully cover the dried cut houseleek forming an extraction phase. In the present example a suitable amount of ethanol (94 wt.-%) is used to fully cover the dried cut houseleek (*sempervivum tectorum L*.) leaves in the flask.
   The extraction can be conducted at any suitable temperature in the range of 5 °C to 100 °C, more preferably 10 °C to 80 °C, even more preferably 15 °C to 60 °C, even more preferably 18 °C to 40 °C. In the present example the extraction is conducted at room temperature (e.g., between 20 to 25 °C).
   The extraction can further be conducted by (occasionally) manually and/or mechanically (magnetic stirrer) stirring the extraction phase and/or using an apparatus facilitating circulation of the extraction agent preferably by use of a pump. In the present case the extraction phase is manually stirred at least three times per day.
   Dependent on the extraction method (temperature, stirring, circulation of extraction agent, etc.) used, the extraction time may range from at least 1 hour to 100 hours, preferably 6 hours to 72 hours, more preferably 12 hours to 48 hours, more preferably 20 to 30 hours. In the present example the extraction is conducted for 24 hours.
   Dependent on the production amount extraction may be conducted two, three or more times.
d) Optionally the remaining solid contents of the houseleek (*sempervivum tectorum L*.), preferably mainly comprising leaves may be separated by suitable means from the resulting extraction phase after finalizing the extraction of step c).
   In the present example the remaining solid contents of the houseleek (*sempervivum tectorum L.*)*,* preferably comprising houseleek (*sempervivum tectorum L.*) leaves are separated by use of a suitable folded filter.
e) Optionally, at least part of the extraction agent in the houseleek *(sempervivum tectorum L.*) extract, preferably houseleek (*sempervivum tectorum L.*) extract of step c) is evaporated by suitable means after finalizing the extraction to produce the inventive houseleek (*sempervivum tectorum L.*) extract concentrate and/or the inventive houseleek (*sempervivum tectorum L.*) leaf extract concentrate.
   In the present example the ethanol as extraction agent is at least partly evaporated by use of suitable means, e.g., a rotary evaporator (rotavap), Büchi / Heidolph. The temperature of the water bath is preferably in the range of 50 °C to 70 °C. The evaporation is preferably conducted until essentially no extraction agent is evaporated anymore (Büchi / Heidolph manually: 60 to 70 mbar; Heidolph Tauto: 35 to 45 mbar).
   Dependent on the production amount evaporation may be conducted two, three or more times.
   Optionally, the produced houseleek (*sempervivum tectorum L.*) extract concentrate, preferably houseleek (*sempervivum tectorum L.*) leaf extract concentrate can further be filtered by suitable means. In the present example a suitable filtrating additive, such as Celite Hyflo super cel (diatomite) is added to the resulting houseleek (*sempervivum tectorum L.*) extract concentrate, preferably houseleek (*sempervivum tectorum L.*) leaf extract concentrate, the concentrate is cooled down and subsequently filtered with a suitable filter paper.
f) A suitable storing additive selected from the group consisting of glycerin, propylene glycole, polysorbate, sorbitole, hydrogenated castor oil, pentylene glycole, preferably glycerin can be added in a suitable amount to the houseleek (*sempervivum tectorum L.*) extract, preferably houseleek (*sempervivum tectorum L.*) leaf extract of step c) or can be added to the houseleek (*sempervivum tectorum L.*) leaf extract concentrate, preferably houseleek (*sempervivum tectorum L*.) leaf extract concentrate during or after evaporation in step d).

In the present case glycerin (BIO, > 99,5% (1.26), plant, pharma quality) is added at the end of the evaporation process in step d) to the houseleek (*sempervivum tectorum L*.) extract concentrate, preferably houseleek (*sempervivum tectorum L.*) leaf extract concentrate to produce a 10 fold houseleek (*sempervivum tectorum L.*) glycerin concentrate, preferably houseleek (*sempervivum tectorum L.*) leaf glycerin concentrate (respectively comprising appr. 85 wt.-% glycerin and remaining water).

The present invention is described in the following on the basis of exemplary embodiments, which merely serve as examples and which shall not limit the scope of the present protective right.

### Examples:

### A: Preparation of an inventive houseleek (sempervivum tectorum L.) extract as houseleek (sempervivum tectorum L.) glycerin extract concentrate

a: In the present example 1 kg dried and cut houseleek (*sempervivum tectorum L.*) leaves (houseleek leaves, BIO CH) are weighed out into a suitable flask for facilitating extraction of the cut houseleek (*sempervivum tectorum L.*) leaves.
b: In the present example a suitable amount of ethanol (94 wt.-%) is provided as the extraction agent.
c: The (dried cut) houseleek (*sempervivum tectorum L.*) leaves of step a) are extracted by adding ethanol (94 wt.-%) as the suitable extraction agent of step b) into the flask of step a) forming an extraction phase and extracting the houseleek (*sempervivum tectorum L.*) leaves to form a houseleek extract.
   In the present example the extraction is conducted at room temperature (e.g., between 20 to 25 °C).
   In the present case the extraction phase is manually stirred at least three times per day.
   In the present example the extraction is conducted for 24 hours.
   In the present example the remaining solid contents of the houseleek (*sempervivum tectorum L*.) leaves are separated by use of a suitable folded filter.
d: In the present example the ethanol as extraction agent is at least partly evaporated by use of suitable means, e.g., a rotary evaporator (rotavap), Büchi / Heidolph. The temperature of the water bath is preferably in the range of 50 °C to 70 °C. The evaporation is preferably conducted until essentially no ethanol is evaporated anymore (Büchi / Heidolph manually: 60 to 70 mbar; Heidolph Tauto: 35 to 45 mbar).
   The produced houseleek (*sempervivum tectorum L.*) extract concentrate, preferably houseleek (*sempervivum tectorum L*.) leaf extract concentrate was further filtered by suitable means. In the present example a suitable filtrating additive Celite Hyflo super cel (diatomite) was added to the resulting houseleek (*sempervivum tectorum L.*) extract concentrate, preferably the houseleek (*sempervivum tectorum L.*) leaf extract concentrate, the concentrate was cooled down and subsequently filtered with a suitable filter paper.
e: Subsequently, glycerine (BIO, > 99.5% (1.26), plant, pharma quality) as the suitable storing additive was added in a suitable amount at the end of the evaporation process in step d) to the houseleek (*sempervivum tectorum L*.) extract concentrate, preferably houseleek (*sempervivum tectorum L.*) leaf extract concentrate to produce a 10 fold houseleek (*sempervivum tectorum L.*) leaf glycerin concentrate, preferably houseleek (*sempervivum tectorum L.*) leaf glycerin concentrate (comprising appr. 85 wt.-% glycerin and remaining water).

### B: Determining dermatological activities of the inventive houseleek (sempervivum tectorum L.) extract

With respect to the biological experiments described in the following the 10 fold houseleek (*sempervivum tectorum L*.) glycerin extract concentrate produced in accordance with Part A hereinbefore has been used. The respective concentrations of 0.1 wt.-%, 0.2 wt.-% and 1 wt.-% of the inventive houseleek (*sempervivum tectorum L*.) extract refer to 0.1 wt.-%, 0.2 wt.-% and 1 wt.-% of the inventive houseleek (*sempervivum tectorum L.*) glycerin extract concentration based on the total weight of the respective cell buffer solutions. The Figures 1 through 8 refer to the inventive houseleek (*sempervivum tectorum L.*) extract 0.1 wt.-%, 0.2 wt.-% and 1 wt.-% by use of the term "HL Extract 0.1%", "HL Extract 0.2 %" and "HL Extract 1.0%".

### B.1 Inventive houseleek (sempervivum tectorum L.) extract activates Peroxisome Proliferator-activated Receptors α and γ (PPARα and PPARγ)

Peroxisome proliferator-activated receptors PPARα and PPARγ are transcription factors that belong to the nuclear hormone receptor family. PPARα seems to contribute to the skin barrier function, activation of β-oxidation of fatty acids, regulation of inflammation, and to play a role in cell differentiation, and proliferation. PPARγ activates cell development (proliferation) and lipogenesis.

PPARα and γ transcriptional assays. To determine the effects of the inventive houseleek (*sempervivum tectorum L.*) extract on PPARs transcriptional activity 3T3-L1 cells are transiently co-transfected with the expression vector GAL4-PPARy and GAL4-PPARα respectively and the luciferase reporter vector GAL4-luc using Roti©-Fect (Carl Roth, Karlsruhe, Germany) following manufacturer's instructions. Twenty-four hours after transfection the cells are treated for 6 h either with 0.1 wt.-%, 0.2 wt.-% and 1 wt.-% of the inventive houseleek (*sempervivum tectorum L.*) extract. Protein lysates are prepared in a buffer containing 25mM Tris-phosphate (pH 7.8), 8mM MgCl₂, 1 mM DTT, 1% Triton X-100 and 7% glycerol and luciferase activity is determined using a luciferase assay kit (Promega, Madison, WI, USA), in an Autolumat LB 9510 (Berthold) following the instructions of the luciferase assay kit (Promega, Madison, WI, USA). The protein concentration in the cell extracts is measured by the Bradford method (BioRad). The RLU/µg is calculated and the results are expressed as percentage of induction of GAL4-Luc activity. Specific transactivation is expressed as fold induction over the control (untreated cells).

The data for PPARα retrieved for the inventive houseleek (*sempervivum tectorum L*.) extract for 0.1 wt.-%, 0.2 wt.-% and 1 wt.-% in comparison with the control of untreated cells are displayed in Figure 1a). Accordingly, the results indicate an agonistic activity on PPARα.

Topical treatment with a PPARα agonist showed a positive effect on atopic dermatitis and stimulated sebaceous lipid production. PPARα activators or compounds that positively regulate PPARα gene expression may, thus, support counteracting ageing processes and may reduce the adverse effects of UVB. According to the experimental data, the inventive houseleek (*sempervivum tectorum L.*) extract seems to be effective in supporting anti-ageing processes as well as in sun care products. Furthermore, the inventive houseleek extract seems to be effective for improving skin moisturizing processes.

The data for PPAR γ retrieved for the inventive houseleek (*sempervivum tectorum L*.) extract for 0.1 wt.-%, 0.2 wt.-% and 1 wt.-% in comparison with the control of untreated cells is displayed in Figure 1b). Accordingly, the results indicate an agonistic activity on PPARγ.

PPARγ agonists are known to provide anti-fibrotic properties, which is characterized by inhibition of pulmonary myofibroblast differentiation and collagen production. These effects are in part induced by transforming growth factor β, leading to an activation of fibroblasts with increased collagen production and expression of cell surface receptors for growth factors. According to the experimental data, the inventive houseleek (*sempervivum tectorum L.*) extract seems to be effective for counteracting wrinkles and supporting refirming effects.

### B.2 Inventive houseleek (sempervivum tectorum L.) extract inhibits Transient receptor Potential cation channel subfamily V member 1 (TrPV1)

TRP channels are involved in cell homeostasis, survival and proliferation, endocrine and exocrine secretory processes and immune as well as inflammatory mechanisms. TRP channels are stimulated by external environmental factors like UV light, cold, heat, air pollution and chemical substances. They play a role in the recognition and physiology of sensitive skin. TrPV1, is among other stimuli activated by capsaicin. TrPV1 inhibitors are described to provide skin-aging preventing properties, wrinkle improvement and skin-whitening features.

TRPV1 antagonistic activity. 293T-VR1 cells (or parental 293T cells as control) are cultured in triplicate in 96-well microtiter plates, the cells are incubated with the 0.1 wt.-%, 0.2 wt.-% and 1 wt.-% of the inventive houseleek (*sempervivum tectorum L.*) extract, for antagonistic activity for 30 min and then stimulated with the agonist Capsaicin (1 µM), for 3 h. Capzasepine (10 µM) is used as the positive control of TRPV-1 antagonism. Then, the cellular viability is measured after 3 h of stimulation by IncuCyte™ Live-Cell Imaging Systems.

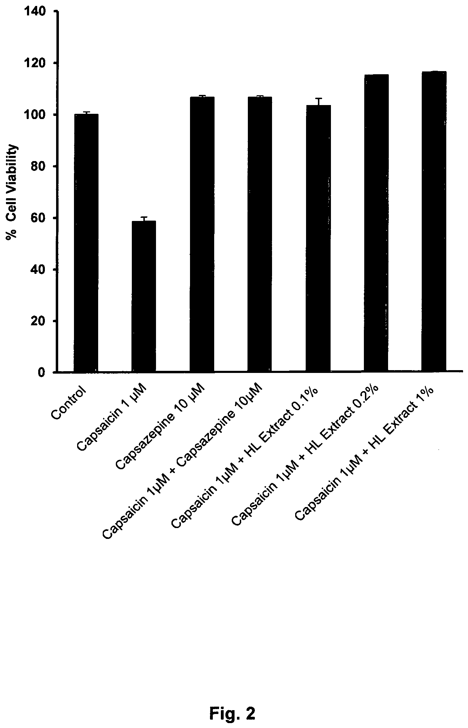

The data retrieved for the inventive houseleek (*sempervivum tectorum L.*) extract for 0.1 wt.-%, 0.2 wt.-% and 1 wt.-% as well as for the positive control of 10 µM Capsazepin are displayed in Figure 2. All concentrations showed an TRPV-1 antagonism. In particular the concentrations of ≥ 0.2 wt.-% inventive houseleek (*sempervivum tectorum L*.) extract showed an improved antagonistic effect in comparison with the positive control of 10 µM Capsazepin.

The research data, thus, indicate that the inventive houseleek (*sempervivum tectorum L.*) extract may be beneficial in the treatment of wrinkles or fine lines and/or sensitive skin.

### B.3 Inventive houseleek (sempervivum tectorum L.) extract increases glucose uptake in keratinocytes

Glucose uptake in keratinocytes. HaCaT cells (5x10⁴) are seeded in 96-well black plates and incubated for 24h. Then, medium is removed and the cells cultivated in OptiMEM, labeled with 50µM 2-NBDG (2-[N-(7-nitrobenz-2-oxa-1,3-diazol-4-yl) amino]-2-deoxy-D-glucose and treated with 0.1 wt.-%, 0.2 wt.-% and 1 wt.-% of the inventive houseleek (*sempervivum tectorum L*.) extract or the positive control with 80mM Rosiglitazone for 24 h. Medium is removed and the wells are carefully washed with PBS and incubated in PBS (100µl/well). Finally the fluorescence is measured using the Incucyte FLR software, the data are analyzed by the total green object integrated intensity (GCUxµm²xWell) of the imaging system IncuCyte HD (Essen BioScience). The fluorescence of Rosiglitazone is taken as 100% of glucose uptake, and the glucose uptake is calculated as (% Glucose uptake) = 100(T - B)/(R - B), where T (treated) is the fluorescence of test substance-treated cells, B (Basal) is the fluorescence of 2-NBDG cells and P (Positive control) is the fluorescence of cells treated with Rosiglitazone.

The data retrieved for the inventive houseleek (*sempervivum tectorum L.*) extract for 0.1 wt.-%, 0.2 wt.-% and 1 wt.-% as well as for the positive control of 80 µM Rosiglitazone are displayed in Figure 3. In particular the concentrations of ≥ 0.2 wt.-% inventive houseleek (*sempervivum tectorum L.*) extract showed an improved glucose uptake in keratinocytes in comparison with the positive control of 80 µM Rosiglitazone. The 0.2 wt.-% inventive houseleek (*sempervivum tectorum L.*) extract concentration stimulates the glucose uptake significantly more (appr. 30.4%) than positive control. The 1 wt.-% inventive houseleek (*sempervivum tectorum L.*) extract significantly increases glucose uptake up to 130.4% in comparison to the positive control. These results may be useful in reversing aged skin conditions to promote healthy regenerated skin.

### B.4 Inventive houseleek (sempervivum tectorum L.) extract inhibits Hypoxia-Inducible Factor 1α (HIF-1α) activity

High levels of transcriptional regulator hypoxia-inducible factor 1 alpha (HIF-1α) are expressed in epidermis which could reflect its important role in local and systemic adaptation to environmental stresses. HIF-1α is an essential mediator of O₂ homeostasis and regulates cutaneous angiogenesis, it controls inflammatory and innate immune responses, modulates skin responses to sunlight by affecting the DNA repair machinery, apoptosis, and the tumorigenic processes.

HIF-1α pathway in fibroblasts and keratinocytes. The NIH3T3-EPO-luc and HaCaT-EPO-Luc cells have been stably transfected with the plasmid Epo-Luc plasmid. The EPO-Hypoxia Response Element (HRE)-luciferase reporter plasmid contains three copies of the HRE consensus sequence from the promoter of the erythropoietin gene in the pGL3 vector. Cells (1x10⁴ for NIH3T3-EPO-luc and 25x10³ for HaCaT-EPO-Luc) are seeded the day before the assay. The next day, the cells are stimulated either with 0.1 wt.-%, 0.2 wt.-% and 1 wt.-% of the inventive houseleek (*sempervivum tectorum L.*) extract or Desferrioxamine (DFX) 150 µM as a positive control. After six hours of stimulation the cells are lysed in 25 mM Tris-phosphate pH 7.8, 8 mM MgCl₂, 1 mM DTT, 1% Triton X-100, and 7% glycerol during 15 min at RT in a horizontal shaker. Luciferase activity is measured using a GloMax 96 microplate luminometer (Promega) following the instructions of the luciferase assay kit (Promega, Madison, WI, USA). The RLU is calculated and the results are expressed as percentage of inhibition induction/inhibition of EPO-luc activity. The experiments for each concentration of the test items have been conducted in triplicate wells.

The data retrieved for the inventive houseleek (*sempervivum tectorum L.*) extract for 0.1 wt.-%, 0.2 wt.-% and 1 wt.-% as well as for the positive control of 150 µM Desferrioxamine are displayed in Figures 4a) (fibroblasts) and 4b) (keratinocytes). All concentrations of the inventive houseleek (*sempervivum tectorum L.*) extract showed an improved inhibition of the HIF-1α activation in fibroblasts and keratinocytes.

Accordingly, the test results suggest that the inventive houseleek (*sempervivum tectorum L.)* extract is effective in reversing aged skin conditions and cellulite skin conditions and in promoting a refirming skin process.

### B.5 Inventive houseleek (sempervivum tectorum L.) extract inhibits tyrosinase activity in B16 melanoma cells

Measurements of Tyrosinase Activity. To measure the tyrosinase activity of the cells, the B16 cells treated for 3 days with 0.1 wt.-%, 0.2 wt.-% and 1 wt.-% of the inventive houseleek (*sempervivum tectorum L.*) extract are lysed by incubation at 4 °C for 30 min in lysis buffer (20 mM sodium phosphate, pH 6.8, 1% Triton X-100, 1 mM PMSF, 1 mM EDTA) containing protease inhibitors cocktail. The lysates are centrifuged at 15,000x g for 10 min to obtain the supernatant as the source of tyrosinase. The reaction mixture contained 20 mM phosphate buffer, pH 6.8, 1.25 mM L-dopa (Sigma-Aldrich). After incubation at 37 °C for 2 h, dopachrome formation is monitored by measuring absorbance at a wavelength of 475 nm in a microplate reader (TECAN). Kojic acid is used as a positive control.

The data retrieved for the inventive houseleek (*sempervivum tectorum L.*) extract for 0.1 wt.-%, 0.2 wt.-% and 1 wt.-% as well as for the positive control of 2 mM Kojic Acid are displayed in Figure 5). All concentrations of the inventive houseleek (*sempervivum tectorum L.*) extract showed a tyrosinase inhibition. In particular concentrations of ≥ 0.2 wt.-% inventive houseleek (*sempervivum tectorum L.*) extract showed an improved tyrosinase inhibition in comparison with the positive control of 2 mM Kojic Acid.

Accordingly, the test results suggest that the inventive houseleek (*sempervivum tectorum L.*) extract is effective in skin lightening and/or skin whithening products. Due to the further biological effects, the inventive houseleek (*sempervivum tectorum L.*) extract seems to be beneficial for promoting skin lightening / skin whitening in particular for sensitive and /or sun exposed skin.

### B.6 Inventive houseleek (sempervivum tectorum L.) extract inhibits melanin synthesis activity in B16 melanoma cells

Determination of Melanin Content and cytotoxicity in B16 cells. To analyse the effect of 0.1 wt.-%, 0.2 wt.-% and 1 wt.-% of the inventive houseleek extract on melanogenesis, B16 melanoma cells are used as a cellular assay system to evaluate the depigmenting activity in cell cultures. Mouse B16 melanoma cells (4A5) obtained from ATCC are treated for 3 days with 0.1 wt.-%, 0.2 wt.-% and 1 wt.-% of the inventive houseleek (*sempervivum tectorum L.*) extract. Then, the cell are washed with phosphate buffer saline (PBS) at the end of the treatment and dissolved in 1 N NaOH containing 10% DMSO for 1 h at 60 °C. The absorbance at 405 nm is measured (TECAN Genious), and the melanin content measured using the authentic standard of synthetic melanin (Sigma-Aldrich, St Louis, MO, USA). Cytotoxicity is measured in parallel by IncuCyte™ Live-Cell Imaging Systems to discard that melanin inhibition is mediated by masked by cytotoxicity. Kojic acid is used as a positive control.

The data retrieved for the inventive houseleek extract for 0.1 wt.-%, 0.2 wt.-% and 1 wt.-% as well as for the positive control of 2 mM Kojic Acid are displayed in Figure 6. All concentrations of the inventive houseleek (*sempervivum tectorum L.*) extract showed an improved melanin synthesis inhibition in comparison with the positive control of 2 mM Kojic Acid.

Accordingly, the test results suggest that the inventive houseleek (*sempervivum tectorum L.*) extract is effective in skin lightening and/or skin whitening products. Due to the further biological effects, the inventive houseleek (*sempervivum tectorum L.*) extract seems to be beneficial for promoting skin lightening / skin whitening in particular for sensitive and /or sun exposed skin.

### B.7 Inventive houseleek (sempervivum tectorum L.) extract activates CannaBinoid receptor type 1 (CB1) in 293T-CB1 cells

CB1 functional assay. 293T-CB1 cells (stably transfected with CB1 cDNAs) are incubated in 24-well plates (1x10⁵ cell/ml) and transiently transfected with 0.1 µg/ml of the plasmid CRE-luc that contains six consensus cAMP responsive elements (CRE) linked to firefly luciferase. Transient transfections are performed with Rotifect (Carl Roth GmbH, Karlsruhe, Germany) according to the manufacturer's instructions and harvested 24 h after transfection. For agonistic activity the transfected cells are stimulated either with increasing concentrations of the inventive houseleek (*sempervivum tectorum L.*) extract produced in part A hereinbefore or with WIN-55, 212-2 (IUPAC name for WIN: - (+)-[2,3-Dihydro-5-methyl-3-(4-morpholinylmethyl)pyrrolo[1,2,3-de]-1,4-benzoxazin-6-yl]-1-napthalenylmethanone, positive control for CB1), during 6 h and then luciferase activity was measured in the cell lysates. CB1 agonist is reflected by the induction of CRE-Luc activity.

The measured data retrieved for the inventive houseleek (*sempervivum tectorum L*.) extract for 0.1 wt.-%, 0.2 wt.-% and 1 wt.-% as well as for the positive control of 1 µM WIN are displayed in Figure 7. In comparison with the positive control WIN-55, 212-2 the inventive houseleek (*sempervivum tectorum L.*) extract showed for concentrations of ≥ 0.2 wt.-% based on the weight of the buffer solution an increased agonistic activity on CB1.

The cannabinoid signalling pathway is recognized for regulating the permeability barrier and epidermal differentiation. In keratinocytes the activation of CB1 supports the epidermal barrier homeostasis and attenuate Th2-type allergic inflammatory responses. A study conducted on CB1 agonists mentions also a mast cell stabilizing function supporting the reduction of itching and histamine secretion (Ständer S, Reinhardt HW, Luger TA, Topische Cannabinoidagonisten, Der Hautarzt, September 2006, Volume 57, Issue 9, pp 801-807).

The agonistic activity on CB1 in keratinocytes indicates that the inventive houseleek (*sempervivum tectorum L.*) extract may be effective when treating sensitive skin.

## Claims

1. Non-therapeutic use of a houseleek (*sempervivum tectorum L.*) extract in a cosmetic formulation, **characterized in that** the houseleek (*sempervivum tectorum L.*) extract is used for enhancing an anti-ageing process in skin, enhancing an anti-wrinkle process in skin, for enhancing a refirming process in skin, for enhancing an anti-cellulite process in skin, and/or for enhancing a regeneration process in skin.

2. A cosmetic formulation for use in enhancing skin condition of sensitive skin comprising
a) an effective amount of houseleek (*sempervivum tectorum L.*) extract for enhancing skin condition of sensitive skin and
b) one, two, three or more cosmetically acceptable carriers, excipients and/or additives.

3. The cosmetic formulation for use according to claim 2, further **characterized in that** the houseleek (*sempervivum tectorum L.*) extract is obtainable by the preparation process of claim 4 or 5 and comprises a suitable amount of a storing additive agent selected from the group consisting of glycerin, propylene glycole, polysorbate, sorbitole, hydrogenated castor oil, or pentylene glycole.

4. A process of preparing a houseleek (*sempervivum tectorum L.*) extract for use in cosmetic formulations, **characterized in that** the process comprises or consists of the following steps:
a) providing a suitable amount of houseleek (*sempervivum tectorum L.*)*,*
b) providing a suitable amount of extraction agent,
c) forming an extraction phase by combining the houseleek (*sempervivum tectorum L.*) of step a) with the extraction agent of step b), extracting the houseleek and providing the houseleek (*sempervivum tectorum L.*) extract,
d) optionally separating by suitable means at least part of the remaining solid content of the houseleek (*sempervivum tectorum L.*) from the extraction phase of step c) after finalizing the extraction,
e) optionally evaporating by suitable means at least part of the extraction agent in step c) after finalizing the extraction to produce a houseleek (*sempervivum tectorum L.*) extract concentrate and
f) adding a suitable amount of a storing additive agent selected from the group consisting of glycerin, propylene glycole, polysorbate, sorbitole, hydrogenated castor oil, or pentylene glycole, to the produced houseleek (*sempervivum tectorum L.*) extract of step c) or d) or houseleek (*sempervivum tectorum L*.*)* extract concentrate of step e).

5. Process of preparing a houseleek extract in accordance with claim 4, wherein the extraction agent of step b) comprises water and/or one, two, three or more suitable extraction alcohols, preferably ethanol.

6. A houseleek (*sempervivum tectorum L.*) extract or houseleek (*sempervivum tectorum L.*) extract concentrate for use in cosmetic formulations obtainable by claim 4 or 5, wherein the houseleek (*sempervivum tectorum L*.) extract or houseleek (*sempervivum tectorum L*.) extract concentrate comprises a suitable amount of a storing additive agent selected from the group consisting of glycerin, propylene glycole, polysorbate, sorbitole, hydrogenated castor oil or pentylene glycole.

## Patentansprüche

1. Nicht-therapeutische Verwendung eines Hauswurz-Extrakts (*sempervivum tectorum L.*) in einer kosmetischen Formulierung, **dadurch gekennzeichnet, dass** der Hauswurz-Extrakt (*sempervivum tectorum L.*) zur Verbesserung eines Anti-Aging-Prozesses in der Haut, zur Verbesserung eines Anti-Falten-Prozesses in der Haut, zur Verbesserung eines Hautstraffungsprozesses in der Haut, zur Verbesserung eines Anti-Cellulite-Prozesses in der Haut und/oder zur Verbesserung eines Regenerationsprozesses in der Haut verwendet wird.

2. Kosmetische Formulierung zur Verwendung bei der Verbesserung eines Hautzustandes empfindlicher Haut, umfassend
a) eine wirksame Menge an Hauswurz-Extrakt (*sempervivum tectorum L*.) zur Verbesserung des Hautzustandes empfindlicher Haut und
b) ein, zwei, drei oder mehr kosmetisch akzeptable Träger, Hilfsstoffe und/oder Zusatzstoffe.

3. Kosmetische Formulierung zur Verwendung nach Anspruch 2, ferner **dadurch gekennzeichnet, dass** der Hauswurz-Extrakt (*sempervivum tectorum L.*) durch das Herstellungsverfahren nach Anspruch 4 oder 5 erhältlich ist und eine geeignete Menge eines Zusatzstoffes für die Lagerung umfasst, ausgewählt aus der Gruppe bestehend aus Glycerin, Propylenglykol, Polysorbat, Sorbitol, hydriertem Rizinusöl oder Pentylenglykol.

4. Verfahren zur Herstellung eines Hauswurz-Extrakts (*sempervivum tectorum L.*) zur Verwendung in kosmetischen Formulierungen, **dadurch gekennzeichnet, dass** das Verfahren die folgenden Schritte umfasst oder aus ihnen besteht:
a) Bereitstellung einer geeigneten Menge an Hauswurz (*sempervivum tectorum L.*)*,*
b) Bereitstellen einer geeigneten Menge an Extraktionsmittel,
c) Bilden einer Extraktionsphase durch Kombinieren des Hauswurzes (*sempervivum tectorum L.*) aus Schritt a) mit dem Extraktionsmittel aus Schritt b), Extrahieren des Hauswurzes und Bereitstellen eines Hauswurz-Extrakts (*sempervivum tectorum L*.)*,*
d) gegebenenfalls nach Abschluss der Extraktion Trennen mindestens eines Teils des verbleibenden Feststoffgehalts des Hauswurzes (*sempervivum tectorum L.*) mit geeigneten Mitteln von der Extraktionsphase aus Schritt c),
e) gegebenenfalls nach Abschluss der Extraktion Verdampfen mindestens eines Teils des Extraktionsmittels aus Schritt c) mit geeigneten Mitteln zur Herstellung eines Hauswurz-Extraktkonzentrats (*sempervivum tectorum L*.) und
f) Zugabe einer geeigneten Menge eines Zusatzstoffes zur Lagerung, ausgewählt aus der Gruppe bestehend aus Glycerin, Propylenglykol, Polysorbat, Sorbitol, hydriertem Rizinusöl oder Pentylenglykol, zu dem erzeugten Hauswurz-Extrakt (*sempervivum tectorum L.*) aus Schritt c) oder d) oder dem Hauswurz-Extraktkonzentrat (*sempervivum tectorum L.*) aus Schritt e).

5. Verfahren zur Herstellung eines Hauswurz-Extraktes nach Anspruch 4, wobei das Extraktionsmittel aus Schritt b) Wasser und/oder ein, zwei, drei oder mehr geeignete Extraktionsalkohole, vorzugsweise Ethanol, umfasst.

6. Hauswurz-Extrakt (*sempervivum tectorum L*.) oder Hauswurz-Extraktkonzentrat (*sempervivum tectorum L.*) zur Verwendung in kosmetischen Formulierungen, erhältlich nach Anspruch 4 oder 5, wobei der Hauswurz (*sempervivum tectorum L*.) Extrakt oder das Hauswurz (*sempervivum tectorum L.*) Extraktkonzentrat eine geeignete Menge eines Zusatzstoffes zur Lagerung ausgewählt aus der Gruppe bestehend aus Glycerin, Propylenglykol, Polysorbat, Sorbitol, hydriertem Rizinusöl oder Pentylenglykol, umfasst.

## Revendications

1. Utilisation non thérapeutique d'un extrait de joubarbe des toits (*sempervivum tectorum L.*) dans une formulation cosmétique, **caractérisée en ce que** l'extrait de joubarbe des toits (*sempervivum tectorum L.*) est utilisé pour améliorer un processus anti-âge dans la peau, améliorer un processus antirides dans la peau, améliorer un processus raffermissant dans la peau, améliorer un processus anticellulite dans la peau et/ou améliorer un processus de régénération dans la peau.

2. Formulation cosmétique destinée à être utilisée pour améliorer les problèmes cutanés des peaux sensibles, comprenant
a) une quantité efficace d'extrait de joubarbe des toits (*sempervivum tectorum L*.) en vue d'améliorer les problèmes cutanés des peaux sensibles et
b) un, deux, trois ou plus de supports, excipients et/ou additifs acceptables sur le plan cosmétique.

3. Formulation cosmétique destinée à être utilisée selon la revendication 2, **caractérisée en outre en ce que** l'extrait de joubarbe des toits (*sempervivum tectorum L.*) peut être obtenu par le procédé de préparation décrit à la revendication 4 ou 5 et comprend une quantité suffisante d'un additif de conservation choisi dans le groupe constitué par la glycérine, le propylène glycol, le polysorbate, le sorbitol, l'huile de ricin hydrogénée ou le pentylène glycol.

4. Procédé de préparation d'un extrait de joubarbe des toits (*sempervivum tectorum L.*) destiné à une utilisation dans des formulations cosmétiques, **caractérisé en ce que** le procédé comprend ou consiste en les étapes suivantes :
a) obtention d'une quantité suffisante de joubarbe des toits (*sempervivum tectorum L.*)*,*
b) obtention d'une quantité suffisante d'agent d'extraction,
c) formation d'une phase d'extraction consistant à associer la joubarbe des toits (*sempervivum tectorum L.*) de l'étape a) à l'agent d'extraction de l'étape b), à extraire la joubarbe des toits et à obtenir l'extrait de joubarbe des toits (*sempervivum tectorum L.*)*,*
d) séparation le cas échéant, par des moyens appropriés, d'au moins une partie du contenu solide restant de la joubarbe des toits (*sempervivum tectorum L.*) de la phase d'extraction de l'étape c) après finalisation de l'extraction,
e) évaporation le cas échéant, par des moyens appropriés, d'au moins une partie de l'agent d'extraction de l'étape c) après finalisation de l'extraction dans le but de produire un concentré d'extrait de joubarbe des toits (*sempervivum tectorum L.*) et
f) ajout d'une quantité suffisante d'un additif de conservation choisi dans le groupe constitué par la glycérine, le propylène glycol, le polysorbate, le sorbitol, l'huile de ricin hydrogénée ou le pentylène glycol à l'extrait de joubarbe des toits (*sempervivum tectorum L*.) produit à l'étape c) ou d) ou au concentré d'extrait de joubarbe des toits (*sempervivum tectorum L*.) obtenu à l'étape e).

5. Procédé de préparation d'un extrait de joubarbe des toits selon la revendication 4, dans lequel l'agent d'extraction de l'étape b) comporte de l'eau et/ou un, deux, trois ou plus d'alcools d'extraction adaptés, de préférence de l'éthanol.

6. Extrait de joubarbe des toits (*sempervivum tectorum L*.) ou concentré d'extrait de joubarbe des toits (*sempervivum tectorum L.*) destiné à une utilisation dans des formulations cosmétiques, obtenu selon la revendication 4 ou 5, l'extrait de joubarbe des toits (*sempervivum tectorum L.*) ou le concentré d'extrait de joubarbe des toits (*sempervivum tectorum L.*) comprenant une quantité suffisante d'un additif de conservation choisi parmi le groupe constitué par la glycérine, le propylène glycol, le polysorbate, le sorbitol, l'huile de ricin hydrogénée ou le pentylène glycol.
